# EUROPEAN PATENT APPLICATION

(11) **EP 3 444 233 A1**
(43) Date of publication of application: **20.02.2019**
(21) Application number: 17782026.3
(22) Date of filing: 11.04.2017
(51) Int. Cl.: C04B 33/04, C04B 35/00, C04B 35/10, C04B 35/101, C04B 35/632, C04B 35/634, B01J 2/00, C01B 33/26, A61Q 1/12

(54) **CERAMIC SPHERES FROM ALUMINOSILICATES**

(30) Priority: 12.04.2016 CO 16092361
(71) Applicant: Suministros de Colombia S.A.S., Medellín (CO)
(72) Inventor: AGUILERA GÁLVEZ, Gabriel Felipe, Medellín (CO); ZAPATA AGUDELO, Nelson Jovanny, Medellín (CO); BUDDE, Tanja, Medellín (CO)
(74) Representative: ABG Intellectual Property Law, S.L.
(86) International application number: PCT/IB2017/052100
(87) International publication number: WO 2017/178978

(57) **Abstract**

The invention relates to a method for obtaining ceramic spheres from alumino silicates, comprising: dry-milling a percentage of the aluminosilicates and wet-milling the remaining percentage; mixing the aluminosilicates obtained from the dry- and wet-milling processes with a binding additive; granulating same; drying the resulting granules; sieving the resulting granules in order to separate same into sub-groups; and sintering the granules obtained at a temperature between 800° and 1500°C.

## Description

### Field of the invention

The field relates to the manufacture of exfoliating scrubs for the cosmetics industry and in particular to mineral-type scrubs that adjust to new worldwide regulations.

### Description of prior art

In the cosmetic exfoliating scrub market, three types exist: organic biodegradable, organic non-biodegradable, and mineral, of which 89% of them are organic non-biodegradable, generally plastics. The main producers of these types of cosmetic scrubs are the United States, Europe and Australia, who ratified legislation prohibiting the use of non-biodegradable materials and who have themselves set different dates for total replacement of these, 2018 for the United States and 2020 for Europe. It was necessary arriving at this conclusion, given studies made in aquatic ecosystems such as great lakes and seas show that one of the main reasons for marine life mortality was micro-plastics from these organic non-biodegradable scrubs (polyethylene).

Accordingly, two exfoliating scrub materials remain which may be used: organic biodegradables and minerals, each having benefits and drawbacks. For example, organic biodegradables produce a large amount of dust when being incorporated into cosmetic products. It is quite difficult to control shape and size. Being biodegradables, they decompose rapidly and depend on production time frames. On the other hand, mineral scrubs cannot be produced in different colors naturally (a desirable feature in this industry), given they have high levels of abrasiveness, and are not sufficiently spherical or round, which in the end could be harmful to the skin instead of being healing. Amongst the currently known mineral scrubs, we have calcium carbonate, precipitated silica, diatomaceous earth and pearlites, which in addition to the mentioned drawbacks, there is a chance they could absorb toxic substances and therefore become toxic waste carriers.

The present invention groups the benefits found in the mentioned scrubs and excludes almost all drawbacks; benefits such as an ample size range, shapes (spherical and rounded) which in addition stimulate blood circulation through blood vessels and skin flexibility, with a wide variety in colors (natural and artificial), density and porosity.

One of the known methods for obtaining spheres from ceramic materials is proppant production in the oil and gas industry. US 6780804 B2 discloses a ceramic particulate material combined with water forming a mixture, then later forming a spherical pellet mix that are then screened and burned. Proppants having a minimum size of 600 micrometers are obtained.

However, the processes described in prior art do not allow obtaining spheres having smaller particle sizes. Therefore, the subject invention uses wet grinding in the raw material preparation process and a mineral suspension is added thus gaining a technical advantage observed when smaller particle sizes are obtained in comparison to those obtained with dry grinding and adding water.

### Brief description of the drawings

FIG. 1 shows sphere sphericity and roundness criteria.
FIG. 2 shows a scanning electronic microscopy photograph of EXAMPLE 1.

### Brief description of the invention

The invention consists of a method of obtaining ceramic spheres from aluminosilicates comprising dry grinding a percentage of the aluminosilicates and the remaining percentage through wet grinding until obtaining a particle size having a D₉₀ between 1 and 25 micrometers; mixing the aluminosilicates obtained through dry grinding and wet grinding using a binding additive; granulating until obtaining granules having a minimum particle size of 50 micrometers; drying the granules obtained until reaching a humidity level between 0 and 5%; screening the granules obtained in order to separate them in subgroups; sintering the granules obtained at a temperature between 800 and 1500° C.

### Detailed description of the invention

The subject invention discloses a method of obtaining ceramic spheres from aluminosilicates. Ceramic spheres are construed as a particle having a high degree of roundness and sphericity and obtained through an irreversible heat process.

Aluminosilicates are kaolin, calcined kaolin, kaolin clay, calcined kaolin clay and arcillite. They can also comprise minerals containing in their mineral composition kaolinite, montmorillonite, illite, smectite, palygorskite, sepiolite, hectorite, nacrite, dickite, halloysite, attapulgite, muscovite, biotite, chlorite, or any other mineralogical variety from the kaolin, clay and mica groups.

In order to carry out the sphere production method, it is initially necessary to know the humidity values of the aluminosilicates from which said ceramic spheres are to be obtained, which determines the maximum amount of water that the aluminosilicate mix must have. Once the required humidity is determined, the percentage of material to be dry-grinded and the percentage of material to be wet-grinded are determined. In an embodiment, the material that is dry-grinded ranges between 50 and 75%. Preferably, 65% of the mix is dry-grinded.

Step a) in this method consists of dry-grinding a percentage of the aluminosilicates, which implies drying the material; in a preferred embodiment, the material is dried until reaching a humidity level under 20%. A humidity percentage in excess of 20% could entail binding or mill blockage and any accessory equipment. Drying can be done using any equipment or known method by anyone skilled in the art. Once dry, the material is fed to the mill until the percentage of retained particles within the 90^{th} percentile of size distribution (D₉₀) is between 1 and 25 micrometers. Once the percentage of dry-grinding is defined, the percentage of wet-grinding is determined.

Wet grinding consists in dispersing the material at a solids concentration corresponding to the amount of water necessary for the granulation process. Likewise, the material is fed to the mill until the percentage of retained particles within the 90^{th} percentile of size distribution (D₉₀) is between 1 and 25 micrometers. Preferably, D₉₀ for wet grinding ranges between 1 and 15 micrometers.

Special attention should be taken in regards to rheological properties of the suspension generated by wet grinding. It should be preferably conserve the following properties: maintain low viscosity (under 1,000 mPa/s), approximately between 200 and 400 degrees of torsion (torsion degrees), positive thixotropy and pseudoplasticity n<1.

Step b) consists of mixing aluminosilicates obtained by wet grinding and dry grinding in a blender. Preferably, the material obtained by wet grinding is measured out over the material obtained by dry grinding until a homogenous blend is obtained. This process must last for an appropriate time in order for aggregates not to form that would prevent granulation of the blend. Excessive humidity would cause agglomeration of said material in bulky masses that would prevent further granulation and on the contrary, if a water defect exists, granule formation would be prevented. Given the spheres formed in this step must be manipulated later on in the following steps, it is necessary to provide the spheres stability in order for them not to disintegrate during handling. Therefore, a binder is added to the blend (after adding the suspension) or mixed with the suspension thus providing said stability. Among the possible binders are the materials comprising those groups made up of carboxymethylcellulose, polyvinyl alcohol, dextrins, lignosulfonates, polymethacrylates, sodium silicate, vinyl silicate, carboxydiethyl cellulose and combinations thereof.

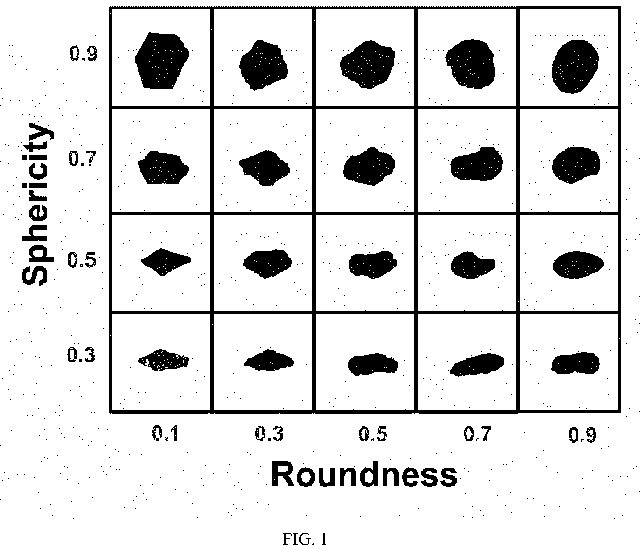

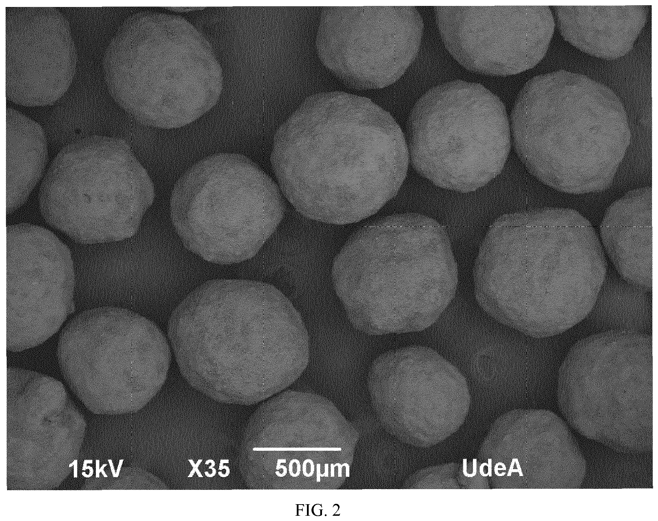

The homogenized material is then taken through step c) comprising granulation until obtaining granules having a minimum particle size of 50 micrometers. For this to happen, the homogenous blend is added to a granulating device; in a preferred embodiment, the granulating device surpasses 15 RPM in the pan and 200 RPM in the rotor, spinning in opposite directions. The opposing movement between the rotor and the pan guarantees granule formation having adequate sphericity and roundness as can be noted in FIG. 1. A short time frame in the granulation step, preferably under 5 minutes in addition to the humidity of the blend's design guarantees a sphericity between 0.7 and 0.9, and a roundness between 0.7 and 0.9. In FIG. 2, the shape of the granules transformed into spheres can be observed using scanning electron microscopy wherein the average particle size is 500 micrometers.

Once the granulation process ends, the granules converted into spheres are unloaded from the device and are taken to a drying step d). Drying can be carried out using any equipment or known methods by anyone skilled in the art, until humidity between 0% and 5% is reached. In a preferred embodiment, humidity must be 0%. Humidity control avoids tension during internal material burning that would cause granule breaking.

Size separation is later carried out using a screening process in order to guarantee size uniformity in the desired subgroups. A subgroup refers to a small size distribution range.

Step f) consists of sintering the spheres obtained in step e), which is basically heat transformation of the composition of spheres in a ceramic matrix. Said sintering is done at a temperature ranging between 800° C and 1500° C. Subjecting the spheres to a temperature less than 800° C would not produce the sintering phenomenon and above 1500° C, the mineralogical phases comprising the ceramic matrix do not undergo significant transformations. The temperature not only determines the degree of sintering, but also the development of color, which must be in line with the initial formulation of raw material (aluminosilicates).

A determined color or degree of sintering can be achieved at a lower temperature by using flux additives. These additives are able to lower melting and sintering points of the material. In this manner, ceramic spheres are achieved with the following color spectra:

**Table 1. Color spectra obtained naturally**

| **Color name** | **pantone #** | **R** | **G** | **B** | **HTML** |
|---|---|---|---|---|---|
| **Nude** | 712 UP | 255 | 202 | 157 | FFCA9D |
| **Ivory** | Warm gray 1 UP | 219 | 213 | 205 | DBD5CD |
| **Terra cotta** | 1625 UP | 255 | 168 | 145 | FFA891 |
| **Terra** | 4755 up | 220 | 198 | 187 | DCC6BB |
| **Sand Beach** | 7501 UP | 222 | 203 | 165 | DECBA5 |
| **Rosé** | 7605 UP | 234 | 196 | 190 | EAC4BE |
| **Quartz** | 5035 UP | 230 | 198 | 199 | E6C6C7 |
| **Quartz Rosé** | 4685 UP | 230 | 202 | 180 | E6CAB4 |
| **Dark Blue** | 7545 C | 66 | 85 | 99 | 425563 |

Other range of colors different than those obtained naturally through temperature can be obtained through the use of pigments based on metallic oxides. Accordingly, there is optionally a step g) which comprises pigmenting the ceramic spheres obtained in step f) using metallic oxides and a flux agent at temperatures between 800 and 1300° C, wherein the metallic oxide has a concentration between 1 and 15% by weight and the flux agent has a concentration between 5 and 30% by weight. This pigmentation can be performed before or after step f).

The preferred metallic oxides can be chosen from: in order to obtain a brown color: iron-chromium oxide (Fe-Cr), iron-chromium-zinc (Fe-Cr-Zn), iron-chromium-zinc-alumina (Fe-Cr-Zn-Al); in order to obtain a blue color: cobalt blue-silica oxide (Co-Si), cobalt blue-alumina-zinc (Co-Al-Zn), vanadium blue-zirconium (V-Zr); in order to obtain a green color: Chromium green oxide (Cr), Blue green chromium-cobalt (Co-Cr), Victoria green (Cr-Ca); in order to obtain black: cobalt-chromium-iron black (Co-Cr-Fe), cobalt-iron-manganese black (Co-Fe-Mn), cobalt-iron-manganese-nickel-chromium black (Co-Fe-Mn-Ni-Cr), cobalt-iron black (Co-Fe); in order to obtain yellow: tin-vanadium yellow (Sn-V), praseodymium-zirconium yellow (Zr-Pr), lead-antimony yellow (Pb-Sb); in order to obtain orange: chromium-antimony-titanium orange (Cr-Sb-Ti), in order to obtain gray: molybdenum-alumina gray (Mo-Al), tin-antimony gray (Sn-Sb), tin-antimony-vanadium (Sn-Sb-V), Chromium-free brown (Sn-Sb-V) and in order to obtain pink and crimson: -manganese-alumina pink (Mn-Al), chromium-alumina pink (Cr-Al), chromium-tin pink (Cr-Sn).

The preferred flux agents may be chosen between sodium hydroxide, sodium feldspar, nepheline, potassium oxide, sodium carbonate, potassium carbonate, calcium carbonate, lithium oxide, lithium carbonate, borax and combinations thereof.

Ceramic spheres characterized because of their pigmentation, particle size between 100 and 800 micrometers, specific gravity between 2.40 and 2.80, and water absorption percentage between 4.0 and 40.0 are obtained and which may be used in the cosmetics industry as a scrubbing material in replacement of non-biodegradable or even biodegradable materials.

### Examples

### Example 1. Ivory

Ceramic spheres from kaolin and kaolin clay. 1.5 kg of kaolin clay was dry-ground until reaching a D₉₀ between 20 and 25 µm. A kaolin clay dispersion in water was prepared at 60% of solids and wet-ground until reaching a D₉₀ of 6 µm. 3.5 kg of kaolin were dry-ground until obtaining a D₉₀ between 25 and 30 µm. 3% by weight of a PVA-type binder additive was used. The wet-ground material, the dry-ground material and the binder were mixed in a blender. The suspended material was added to the dry material, measuring out the addition throughout 45 seconds. The mix was performed in a 10 L capacity device, at a rotor speed of 3000 RPM and the pan at 72 RPM. Granulation was carried out for a minute at a rotor speed of 4000 RPM and the pan at 72 RPM. The rotor rotated in opposite direction of the pan. Granules having a roundness of 0.9 and sphericity of 0.9 were obtained. The granules were dried at 100° C during 4 hours, until a final humidity of 0.5% was obtained. The material was screened in order to obtain the following granule specifications.

**Table 2. Size distribution**

| **Screen number** | **Percentage** |
|---|---|
| +M20 | 37% |
| +M30/+M40 | 57% |
| -M40/+M120 | 4% |
| -M120 | 2% |

Granules were sintered at a temperature of 1300° C during one hour. Ivory-colored ceramic spheres were obtained having a water absorption percentage of 22.62% and specific gravity of 2.70.

### Example 2. Nude

The ceramic spheres obtained in EXAMPLE 1 were pigmented at a temperature of 1000° C during one hour. Nude-colored ceramic spheres were obtained having a water absorption percentage of 22.26% and specific gravity of 2.50.

### Example 3. Terra cotta

Ceramic spheres made of kaolin and high iron content arcillite. 1.5 kg of high iron content arcillite was dry-ground until reaching a D₉₀ between 20 and 25 µm. A high iron content arcillite clay dispersion in water was prepared at 65% of solids and wet-ground until reaching a D₉₀ of 8 µm. 3.5 kg of kaolin was dried until obtaining humidity of 5% and then dry-ground until obtaining a D₉₀ between 25 and 30 µm. 2% by weight of a PVA-type binder additive was used. The wet-ground material, the dry-ground material and the binder were mixed in a blender. The suspended material was added to the dry material, measuring out the addition throughout 45 seconds. Then, the binder was added measuring out the addition throughout 45 seconds. The mix was performed in a 10 L capacity device, at a rotor speed of 3000 RPM and the pan at 60 RPM. Granulation was carried out for a minute at a rotor speed of 4000 RPM and the pan at 72 RPM. The rotor rotated in opposite direction of the pan. Granules having a roundness of 0.9, sphericity of 0.7 and 20% humidity were obtained. The granules were dried at 100° C during 4 hours, until a final humidity of 0.5% was obtained. The material was screened in order to obtain the following granule specifications.

**Table 3. Size distribution**

| **Screen Number** | **Percentage** |
|---|---|
| +M20 | 16% |
| -M20/+M40 | 32% |
| -M40/+M70 | 32% |
| -M70/+M120 | 10% |
| -M120 | 10% |

Granules were sintered at a temperature of 1300° C during one hour. Terra cotta-colored ceramic spheres were obtained having a water absorption percentage of 18.61% and specific gravity of 2.52.

### Example 4. Terra

The ceramic spheres obtained in EXAMPLE 3 were pigmented at a temperature of 1100° C during one hour. Terra-colored ceramic spheres were obtained having a water absorption percentage of 10.30% and specific gravity of 2.53.

### Example 5. Sand beach

The ceramic spheres obtained in EXAMPLE 3 were pigmented at a temperature of 1300° C during one hour. Sand beach-colored ceramic spheres were obtained having a water absorption percentage of 4.35% and specific gravity of 2.55.

### Example 6. Rose

Ceramic spheres made of medium iron content arcillite and calcined kaolin. 1.5 kg of medium iron content arcillite was pre-wet-ground at 65% of solids, until reaching a D₉₀ of 18 µm. Thereafter, fine wet-grinding was carried out at 65% of solids until obtaining a D₉₀ of 11 µm. 3.5 kg of previously-calcined kaolin was dry-ground at 0% humidity until obtaining a D₉₉ of 17 µm. 3% by weight of a PVA-type binder additive was used. The wet-ground material, the dry-ground material and the binder were mixed in a blender. The suspended material was added to the dry material, measuring out the addition throughout 45 seconds. Then, the binder was added measuring out the addition throughout 45 seconds. The mix was performed in a 10 L capacity device, at a rotor speed of 3000 RPM and the pan at 72 RPM. Granulation was carried out for 30 seconds at a rotor speed of 4000 RPM and the pan at 72 RPM. The rotor rotated in opposite direction of the pan. Granules having a roundness of 0.9, sphericity of 0.8 and between 21% and 22% humidity were obtained. The granules were dried at 100° C during 4 hours, until a final humidity of 0.5% was obtained. The material was screened in order to obtain the following granule specifications.

**Table 4. Size distribution**

| **Screen Number** | **Percentage** |
|---|---|
| +M20 | 15% |
| -M20/+M40 | 18% |
| -M40/+M70 | 24% |
| -M70/+M120 | 23% |
| -M120 | 17% |

Granules were sintered at a temperature of 800° C during one hour. Rosé-colored ceramic spheres were obtained having a water absorption percentage of 34.00% and specific gravity of 2.50.

### Example 7. Quartz

The ceramic spheres obtained in EXAMPLE 6 were pigmented at a temperature of 900° C during one hour. Quartz-colored ceramic spheres were obtained having a water absorption percentage of 12.26% and specific gravity of 2.51.

### Example 8. Quartz Rose

The ceramic spheres obtained in EXAMPLE 6 were pigmented at a temperature of 1200° C during one hour. Quartz Rosé-colored ceramic spheres were obtained having a water absorption percentage of 26.05% and specific gravity of 2.5.

### Example 9. Dark blue

A dark blue-colored pigmentation of EXAMPLE 2 wherein a 5% cobalt silicate, 3% sodium silicate and 10% water pigment solution is used. The cobalt silicate is heat transformed into the metallic oxide, cobalt oxide. The cobalt silicate, sodium silicate and water solution was added to the ceramic spheres using a rotor speed of 1500 RPM and a pan speed of 15 RPM, until a homogenous sphere color was obtained. The pigmented ceramic spheres were dried at 100° C during 4 hours. They were again sintered at 1200° C. Dark blue-colored ceramic spheres are obtained.

## Claims

1. A method for obtaining ceramic spheres from aluminosilicates comprising:
a) dry-grinding a percentage of the aluminosilicates and wet-grinding the remaining percentage until obtaining a particle size having a D₉₀ between 1 and 25 micrometers;
b) mixing the aluminosilicates obtained by dry-grinding and wet-grinding in step a) with a binding additive;
c) granulating until obtaining granules having a minimum particle size of 50 micrometers;
d) drying the granules obtained in step c) until reaching a humidity between 0 and 5%;
e) screening the granules obtained in step d) in order to separate them into subgroups;
f) sintering the granules obtained in step e) at a temperature between 800° and 1500° C.

2. The method according to Claim 1, wherein in step a), the aluminosilicates are kaolin, calcined kaolin, kaolin clay, calcined kaolin clay and arcillite.

3. The method according to Claim 1, wherein in step a), the dry-grinding comprises between 50 and 75% of the blend.

4. The method according to Claim 1, wherein optionally there is a step g), comprising pigmenting the ceramic spheres obtained in step f) by using metallic oxides and a flux agent at temperatures between 800° and 1300° C, wherein the metallic oxide has a concentration between 1% and 15% by weight and the flux agent has a concentration between 5% and 30% by weight.

5. The method according to Claim 1, characterized because the granules obtained in step e) are pigmented using metallic oxides and a flux agent at temperatures between 800° and 1300° C, wherein the metallic oxide has a concentration between 1% and 15% by weight and the flux agent has a concentration between 5% and 30% by weight.

6. The method according to Claim 1, wherein in step b), the binding agent is selected from the group comprising carboxymethylcellulose, polyvinyl alcohol, dextrins, lignosulfonates, polymethacrylates, sodium silicate, vinyl acetate, carboxydiethyl cellulose and combinations thereof.

7. A ceramic sphere characterized because it is pigmented, and having a particle size between 100 and 800 micrometers, a specific gravity between 2.40 and 2.80, and a water absorption percentage between 4.0 and 40.0.
